# EUROPEAN PATENT SPECIFICATION

(11) **EP 1 550 689 B1**
(45) Date of publication and mention of the grant of the patent: **24.04.2013**
(21) Application number: 03784596.3
(22) Date of filing: 08.08.2003
(51) Int. Cl.: C08G 85/00, C08G 61/12, C08J 5/00, A61F 2/08, A61F 2/70, B32B 27/00, C08L 101/00

(54) **PROCESS FOR PRODUCING CONDUCTIVE POLYMER**
VERFAHREN ZUR HERSTELLUNG VON LEITFÄHIGEM POLYMER
PROCEDE DE PRODUCTION D'UN POLYMERE CONDUCTEUR

(30) Priority: 09.08.2002 JP 2002233617; 11.09.2002 JP 2002265859; 02.10.2002 JP 2002289365; 22.10.2002 JP 2002307559; 22.10.2002 JP 2002307472
(43) Date of publication of application: 06.07.2005
(73) Proprietor: Eamex Corporation, Suita-shi, Osaka 564-0062 (JP)
(72) Inventor: ZAMA, Tetsuji, c/o EAMEX CORPORATION, Osaka 564-0062 (JP); HARA, Susumu, c/o EAMEX CORPORATION, Osaka 564-0062 (JP); SEWA, Shingo c/o EAMEX CORPORATION, Osaka 564-0062 (JP)
(74) Representative: Scott, Susan Margaret
(86) International application number: PCT/JP2003/010111
(87) International publication number: WO 2004/014987

(56) References cited:
- EP-A- 0 247 366
- EP-A2- 0 924 033
- JP-A- 2000 336 154
- JP-A- 2001 240 742
- DATABASE WPI Week 199511 Thomson Scientific, London, GB; AN 1995-076615 XP002493712 & ES 2 062 930 A1 (UNIV DEL PAIS VASCO) 16 December 1994 (1994-12-16)
- VILLARREAL I., MORALES E., ACOSTA J.L.: 'Electrochemical synthesis of poly(3-methylthiophene): A kinetic study' JOURNAL OF POLYMER SCIENCE PART B POLYMER PHYSICS vol. 38, no. 9, 01 May 2000, pages 1258 - 1266, XP002973930

## Description

### Field of the Invention

The present invention relates to an electrochemomechanical deformation method using specific conductive polymers.

### Background Art

Conductive polymers such as polypyrrole are known to have electrochemomechanical deformation, phenomena of stretching and deforming by electrochemical redox reaction. Recently, this electrochemomechanical deformation of conductive polymers has been attracting public attention, because this is expected to be applied for the drive of use for, for example, artificial muscles, robot arms, artificial arms and limbs, power suits, and actuators. As a process for producing conductive polymers which show such electrochemomechanical deformation, a producing process by electrochemical polymerization method is common. A common electrochemical polymerization method includes the method in which monomer such as pyrrole is added to an electrolyte solution, followed by applying voltage between a working electrode and a counter electrode.

Conductive polymer forms obtained by electrochemical polymerization can be stretched or deformed by applying voltage to the films. As such conductive polymers that can be stretched, free-standing films of conductive polymers can also be used for the use of artificial muscles, and for example, Japan Unexamined Patent Publications No Hei 11-169393 and No Hei 11-169394 state that artificial muscles with polyaniline films formed on both sides of solid electrolyte can also be used.

In addition, regarding actuators using conductive polymers, composition of actuators provided with electrolyte, counter electrodes, and polypyrrole films in the cell was reported in 1997 (A. Della Santa et al, "Performance and work capacity of a polypyrrole conducting polymer linear actuator" Synthetic Metals, Elsevier Science, 1997,90, P93-100). It is stated that in the state where a polypyrrole film and a couter electrode are immersed in electrolyte, by the voltage application between the counter electrode and the polypyrrole film, the polypyrrole film makes electrochemical deformation and that despite the load of 14.6MPa (45 g) by polypyrrole film, this actuator makes deformation of 1 %. In other words, this actuator can generate electrochemical stress of 14 MPa in the length direction by electrochemomechanical deformation, however, the stretch remains 1%.

However, as practical uses, in operating artificial muscles, robot arms, artificial hands and limbs and the like, one deformation or strain generally makes one movement.

Therefore, in order for artificial muscles and the like to make full movement, large deformation or strain is required per deformation or per strain. When conventional conductive polymers are used as a driving source for practical use, actuator elements capable of making electrochemomechanical deformation, which uses conventional conductive polymers, regarding the amount of deformation obtained by repeating cycles (redox cycles) of expansion and contraction by electrochemomechanical deformation, the amount of deformation or the amount of strain per redox cycle is not satisfactory. Therefore, in order to use conductive polymers for practical use such as for artificial muscles, robot arms or artificial hands, larger amount of deformation or of strain is required per redox cycle when electrochemomechanical deformation is made. For example, conventional conductive polymers which include sodium p-toluenesulfonate as dopant, deformation ratio per redox cycle is small and they can be used as actuators for the use which requires small amount of deformation or of strain. However, in order to use conductive polymers for the use which requires large amount of deformation or of strain per redox cycle such as for artificial muscles, electrochemical strain per redox cycle of conductive polymers should further be enhanced.

In addition, in order to enhance practicality including applications for artificial muscles, it is desirable that the time taken from the order of generating deformation or strain including applying voltage to conductive polymers until the actual generation of desirable amount of deformation that is, strain is short or the electrochemical strain per specific time is large, if possible. In other words, in order to employ conventional conductive polymers for practical use, it is desirable to enhance the ratio of the length of conductive polymer forms in the initial state in a specific time after applying voltage to conductive polymers to the deformed length or electrochemically strained length, in other words, it is desirable to enhance the electrochemical strain per specific time, if possible, in addition to enhancing deformation ratio per redox cycle of conductive polymers since it further enhances practicality.

It is reported in Synthetic Metals, 90 (1997) 93-100 that conductive polymers obtained by electrochemical polymerization as conductive polymers used for artificial muscles, regarding deformation and force generated electrically per redox, when polypyrrole formed as a film has electrochemical strain of 1%, electrochemical stress with about 3 MPa is generated.

Further, when conductive polymers are used for actuators such as micro machines or artificial muscles, which are applied uses, since large strain motion is generated by actuators, electrochemical strain per redox cycle is required to be greatly enhanced from the current level of about 1%. However, relationship between electrochemical strain and electrochemical stress in actuators is inversely proportional. For this reason, in order to increase electrochemical stress which is required to move load added for actuators, electrochemical strain of actuators decreases. Therefore, in actuators which use conventional conductive polymers, stress generated electrically decreases to less than 3MPa when electrochemical strain obtained in redox cycle is set to be more than 1%, which makes it difficult to obtain conductive polymers with well balanced electrochemical strain and stress.

In addition as actuators which use conventional conductive polymers, those using sodium benezenesulfonate or sodium p-toluenesulfonate as dopant are common and actuators whose electrochemical strain per redox cycle is more than 3% have not been obtained. Thus, especially, in order to apply for micro machines or buried artificial muscle which requires to obtain large force with a small size, electrochemical strain or electrochemical stress of actuators using conventional conductive polymers is not satisfactory. For practical use, it is further desirable that actuators obtained by conductive polymers have larger electrochemical strain and stress compared with conventional ones.

The present invention utilises a process for producing conductive polymers having excellent electrochemical strain per redox cycle.

### SUMMARY OF THE INVENTION

The preferred embodiments of the present invention have been developed in view of the above-mentioned and/or other problems in the related art. The preferred embodiments of the present invention can significantly improve upon existing methods and/or apparatuses.

The invention provides an electrochemomechanical deformation method according to claim 1. The invention also provides an actuator according to claim 5.

The invention of the present application utilises a process for producing conductive polymers by an electrochemical polymerization method, wherein said conductive polymers have deformation property by electrochemical redox reaction, said electrochemical polymerization method is a polymerization method using electrolyte including organic compounds as solvents, and wherein said organic compounds include
(1) at least one chemical bond species selected from the group consisting of ether bond, ester bond, carbon-halogen bond, and carbonate bond and/or
(2) at least one functional group selected from the group consisting of hydroxyl group, nitro group, sulfone group, and nitryl group
and wherein said electrolyte used in said polymerisation process includes trifluoromethanesulfonate ions and/or anions in which a plurality of fluorine atoms which are bonded to a central atom.

Conductive polymer actuators obtained by this process have large deformation per redox cycle and can preferably be used for actuator elements which are driving sources for a practical use.

Said electrochemical polymerization method may employ a metal electrode as a working electrode on which conductive polymers are formed.

Since conductive polymers obtained by this process greatly exceed electrochemical strain of 1 % which is the conventional electrochemical strain and can obtain larger stress generated electrically compared with conventional ones, they can be used for actuator elements which are driving sources for a practical use.

Conductive polymer forms obtained by said process are not only excellent in the electrochemical strain per redox cycle, but also excellent in strain per specific time. Therefore, said conductive polymer forms can preferably be used for actuator elements which are driving sources for a practical use.

The invention of the present application also relates to an electrochemomechanical deformation method which comprises deforming conductive polymer forms obtained by said process by electrochemical redox reaction in electrolyte including at least one compound selected from the group consisting of trifluoromethanesulfonate ions, anions in which a plurality of fluorine atoms are bonding to a central atom, and sulfonate whose carbon number is less than 4.

Since in said conductive polymer forms driven by conducting said electrochemomechanical methods, electrochemical strain per redox cycle increases, they can be used for actuator elements which are driving sources for a practical use.

Conductive polymers as described above can be used for laminates which include conductive polymer-containing layers and solid electrolyte layers. When conductive polymers included in said conductive polymer-containing layers are obtained by said process for producing the conductive polymers, said laminates are capable of enhancing electrochemical strain per redox cycle, therefore, they can be used for actuator elements which are driving sources for a practical use.

The invention of the present application also relates to actuators comprising moving parts, counter electrodes, and electrolyte, wherein said actuators use operating portions which include conductive polymers obtained by above mentioned conductive polymers as moving portions. Since said actuators are capable of enhancing electrochemical strain per redox cycle of actuators regarding electrochemomechanical deformation generated by voltage application between counter electrodes and moving parts, they can be used for actuator elements which are driving sources for a practical use.

The actuator of the present application has utility in a positioning device, a posture control device, an elevating device, a carrier device, a moving device, a regulating device, an adjusting device, a guiding device, or a joint device using the above conductive polymer forms as actuator elements for a driving part, or a pressing device using the above conductive polymer forms as actuator elements for a pressing part. In addition, the actuator of the present application relates to a positioning device, a posture control device, an elevating device, a carrier device, a moving device, a regulating device, an adjusting device, a guiding device, or a joint device using the above actuators for a driving part and a pressing device using the above actuators for a pressing part.

### BRIEF DESCRIPTION OF THE DRAWINGS

The preferred embodiments of the present invention are shown by way of example, and not limitation, in the accompanying figures, in which:
Fig. 1 is a perspective view regarding the appearance of an example of the embodiment of actuators of the invention of the present application.
Fig. 2 is a sectional view taken along the line A-A regarding actuators of Fig. 1.

### DETAILED DESCRIPTION OF THE PREFERRED EMBODIMENTS

Hereinafter, explanation on the invention of the present application goes further in detail.

### (Electrolyte)

In the invention of the present application, electrolyte used for electrochemical polymerization includes organic compounds as solvents. Said organic compounds include at least one chemical bond selected from the group consisting of ether bond, ester bond, carbon-halogen bond, and carbonate bond, and/or at least one functional group selected from the group consisting of hydroxyl group, nitro group, sulfone group, and nitryl group. Said organic compounds include at least either one of these chemical bond or these functional groups in a molecule. Said organic compounds may include either of chemical bond selected from the group of said chemical bond or functional groups selected from the group of said functional group. In addition, said organic compounds may include at least one chemical bond selected from said chemical bond group or at least one functional groups selected from the group of said group of functional groups.

As said organic compounds, following groups of organic compounds can be exemplified; 1,2-dimethoxyethane, 1,2-diethoxyethane, tetrahydrofuran, 2-methyltetrahydrofuran, 1,4-dioxane (so far, organic compounds including ether bond), γ-butyrolactone, ethyl acetate, n-butyl acetate, tert-butyl acetate, 1,2-diacetoxyethane, 3-methyl-2-oxazolidinone, methyl benzoate, ethyl benzoate, butyl benzoate, diethyl phthalate (so far, organic compounds including ester bond), propylene carbonate, ethylene carbonate, dimethyl carbonate, diethyl carbonate, methyl ethyl carbonate (so far, organic compounds including carbonate bond), ethylene glycol, butanol, 1-hexanol, cyclohexanol, 1-octanol, 1-decanol, 1-dodecanol, 1-octadecanol (so far, organic compounds including hydroxyl group), nitromethane, nitrobenzene (so far, organic compounds including nitro group), sulfolane, dimethyl sulfone (so far, organic compounds including sulfone group), and acetonitrile, butyronitrile, benzonitrile (so far, organic compounds including nitrile group). In addition, although organic compounds including hydroxyl group are not specifically limited, they are preferably polyalcohol or mono alcohol with a carbon number of not less than 4 for good electrochemical strain. Further, other than said examples, said organic compounds may be organic compounds which include two or more bond or functional groups out of ether bond, ester bond, carbonate bond, hydroxyl groups, nitro groups, sulfone groups, and nitrile groups with any combinations in a molecule. Said organic compounds are preferably organic compounds including ester bond since the electrochemical strains of conductive polymers thus obtained are large.

In addition, in the process for producing conductive polymers when organic compounds included in electrolyte as solvents include carbon-halogen bond, said organic compounds may be halogenated hydrocarbon. Halogenated hydrocarbon is not specifically limited as long as at least one of hydrogen in hydrocarbon is replaced by halogen atom and can stably be present under the condition of electrochemical polymerization as liquid. As said halogenated hydrocarbon, for example, dichloromethane and dichloroethane can be exemplified. One species of said halogenated hydrocarbons can be used as a solvent in said electrolyte, or two or more species can be used together. In addition, said halogenated hydrocarbon can be used with the above mentioned organic compounds as a mixture, and mixed solvents of said organic solvents other than halogenated hydrocarbon can also be used as solvents in said electrolyte.

When said organic compounds are used as solvents of electrolyte by mixing two or more of them, electrochemical strain per redox cycle of conductive polymers obtained by polymerization can be enhanced.

In the process for producing conductive polymer, electrolyte used in electrochemical polymerization includes organic compounds subject to electrochemical polymerization (for example, pyrrole) and trifluoromethanesulfonate ion and/or anions including a plurality of fluorine atoms bonding to a central atom. Conductive polymers obtained by electrochemical polymerization using this electrolyte have electrochemical strain per redox cycle and/or strain per specific time in electrochemomechanical deformation. By the above mentioned electrochemical polymerization, trifluoromethanesulfonate ion and/or anions which include a plurality of fluorine atoms which are bonded to a central atom are taken into the conductive polymers.

Although the content of trifluoromethanesulfonate ion and/or anions which include a pluraility of fluorine atoms which are bonded to a central atom in the electrolyte is not specifically limited, the content is preferably 0.1 to 30% by weight and more preferably 1 to 15% by weight in the electrolyte.

Trifluoromethanesulfonate ion is represented by the chemical formula of CF₃SO₃⁻. Further, anions which include a plurality of fluorine atoms which are bonded to a central atom have structures in which a plurality of fluorine atoms bond to a central atom such as boron, phosphorus, antimony, and arsenic. Although anions which include a plurality of fluorine atoms which are bonded to a central atom are not specifically limited, tetrafluoroborate ion (BF₄⁻), hexafluorophosphate ion (PF₆⁻), hexafluoroantimonate ion (SbF₆⁻), and hexafluoroarsenate ion (AsF₆⁻) can be exemplified. Among them, CF₃SO₃⁻, BF4⁻, and PF₆⁻ are preferable from the view point of safety to human bodies and the like, and CF₃SO₃⁻ and BF₄⁻ are more preferable. Anions which include a plurality of fluorine atoms which are bonded to said central atom may consist of one species, or two or more species thereof may be used in the electrolyte at the same time. Further, trifluoromethanesulfonate ions and anions which include plurality of fluorine atoms which are bonded to a central atom may be used in the electrolyte at the same time.

In the process for producing conductive polymers, electrolyte used in the electrochemical polymerization method includes monomers of conductive polymers in solution. In addition, further, said electrolyte can include other known additives such as polyethylene glycol and polyacrylamide.

In the process for producing conductive polymers, metal electrodes may be used as the working electrode, on which conductive polymers are formed.

### (Metal electrode)

By using a metal electrode in electrochemical polymerization, compared when an electrode whose main material is non-metal such as ITO glass or NESA glass electrodes is used, actuators using conductive polymers obtained can develop larger stress generated electrically. Although said metal electrode is not specifically limited as far as the electrode is mainly made of metal, electrode of a single metal or alloy elements selected from a group consisting of Pt, Ti, Au, Ni, Ta, Mo, Cr and W can preferably be used. As a metal species included in a metal electrode, Ni and Ti are particularly preferable since electrochemical strain and stress generated electrically of conductive polymers obtained by said process are large and since electrodes can easily be obtained.

### (Condition of electrochemical polymerization)

In electrochemical polymerization used in known electrochemical polymerization methods can be used, which include any of constant potential methods, constant current methods, and potential sweep methods. For example, said electrochemical polymerization method can be conducted with current density of 0.01 to 20mA/cm² at a reaction temperature of -70 to 80°C, and preferably, with current density of 0.1 to 2mA/cm² at a reaction temperature of -40 to 40°C for obtaining conductive polymers of preferable film quality, and further preferably, at a reaction temperature of -30 to 30°C. In addition, working electrodes are not specifically limited as long as they can be used for electrochemical polymerization, and ITO glass electrodes or metal electrodes can be used.

Monomers are not specifically limited as long as they are compounds which may be polymerized to show conductivity. For example, five-membered heterocyclic compound such as pyrrole, and thiophene, isothianaphthene, and derivatives thereof such as alkyl group thereof or oxyalkyl group thereof, may be used. Among them, five-membered heterocyclic compounds such as pyrrole, thiophene and derivatives thereof are preferable and in particular, conductive polymers which include pyrrole and/or pyrrole derivatives are preferable for easy manufacturing and stability as conductive polymers. In addition, the above monomer can be used in combinations of two or more of them.

Conductive polymers used in the invention of the present application are not specifically limited as long as they have good stretching property and polypyrrole, polythiophene, polyaniline, polyphenylene films can for example be used. Said conductive polymers preferably include pyrrole and/or pyrrole derivatives in molecular chains for easy manufacturing, stability as conductive polymers, and further for excellent electrochemical deformation property.

It is considered that since said conductive polymers include trifluoromethanesulfonate ions and/or anions which include a plurality of fluorine atoms bonded to a central atom included in electrolyte as dopant, said conductive polymers show excellent electrochemical strain of electrochemomechanical deformation per redox reaction and also show excellent strain per specific time. In addition, when a metal electrode is used as the working electrode at the time of electrochemical polymerization, said conductive polymers show excellent electrochemical strain per redox cycle in electrochemomechanical deformation and further, larger force can be generated by electrochemomechanical deformation.

### (Forms)

Conductive polymers obtained by the above first process can be made into desired shapes, or forms, including the conductive polymers as resin component. Although the shape of said conductive polymer forms is not specifically limited and this may for example be film-like, pipe-shaped, tube-shaped, prismatic, or fibrous, the shape is preferably film-like since said conductive polymer separates out on a working electrode at the time of electrochemical polymerization. In addition, said working electrodes are not specifically limited as long as they can be used for electrochemical polymerization and ITO glass electrodes or metal electrodes can for example be used.

Conventionally, in electrochemomechanical deformation of conductive polymers, when conductive polymers are film-like, the electrochemical strain was only 1% at most per redox cycle in the long-length direction. However, the conductive polymer forms used in the invention of the present application have excellent electrochemical strain of not less than 3% or in particular, not less than 5% per redox cycle in the length direction of conductive polymer forms, by including trifluoromethanesulfonate ions and/or anions which include a plurality of fluorine atoms which are bonded to a central atom, included in the conductive polymer forms as dopant. Conventional conductive polymer forms having small electrochemical strain could be used only for uses not requiring large potential such as switching devices or sensoring machines. On the other hand, conductive polymer forms used in the invention of the present application can be used in applications requiring large electrochemical strain represented by artificial muscles since electrochemical strain is not less than 3% per redox cycle in the length direction. In addition, said conductive polymer forms can appropriately include conductive materials such as metal wires or conductive oxides other than dopant in order to lower the resistivity as a working electrode.

Further, conductive polymer forms deforming with electrochemical redox are also the conductive polymer forms whose electrochemical strain is not less than 3% redox cycle of 20 seconds in the length direction. Said conductive polymer forms whose deformation ratio is not less than 3% per redox cycle of 20 seconds in the length direction are obtained as described above. Compared with conventional conductive polymers, since these conductive polymer forms can realize larger strain of an end portion of conductive polymer forms in a specific time after initiating the voltage application at one point, these conductive polymer forms can be used for actuator elements which are driving sources for the practical use.

When said conductive polymer forms are used as actuators, said actuators can be provided with said conductive polymer forms, counter electrodes, and electrolyte where said actuators can be provided with counter electrodes and electrolyte so that voltage can be applied between said counter electrodes and conductive polymer forms interposing said electrolyte therebetween. Since said actuator has electrochemical strain of not less than 3% per redox cycle of 20 seconds in the length direction by applying voltage from one end of conductive polymer forms, said actuator can be preferable for use with quicker response such as for driving parts of artificial muscles.

Also when a metal electrode is used as a working electrode on which a conductive polymer is formed, the shape of said conductive polymer forms is not specifically limited and it may for example be film-like, pipe-shaped, tube-shaped, prismatic or fibrous. The shape is preferably film-like since said conductive polymer deposits out on a working electrode at the time of electrochemical polymerization. Said film-like material may be formed so that the conductive polymer obtained by the above producing method coats the surface of a material to be coated by a known method.

### (Laminates)

Laminates which include conductive polymer layers and solid electrolyte layers may include the above conductive polymers in said conductive polymer layers.

Since laminates include said conductive polymer layer and said solid electrolyte layer, electrolyte in said conductive polymer layer is provided to said conductive polymer layer and conductive polymers includes in said conductive polymer-containing layer stretch greatly by electrochemical redox reaction, thereby realizing large electrochemical strain per redox cycle at the time of electrochemomechanical deformation. Although it is preferable that said conductive polymer layer and solid electrolyte layer in said laminates contact each other directly, other layers may be interposed therebetween if electrolyte in said solid electrolyte layer can be shifted to said conductive polymers. In addition, said conductive polymer-containing layer can include substrates including for example conductive oxides and metal wires which do not greatly disturb electrochemomechanical deformation.

Although said solid electrolyte is not specifically limited, since it can realize big drive, said solid electrolyte is preferably ion exchange resin. As said ion exchange resin, known ion exchange resin can be used and for example, tradename "Nafion" (perfluorosulfonate resin, manufactures by DuPont) can be used.

When said laminates are used as actuators, actuators can be provided with counter electrodes and said laminates so that voltage is applied between said counter electrodes and conductive polymer-containing layer in said laminates interposing solid electrolyte in said laminates therebetween.

### (Electrochemomechanical deformation method)

The invention of the present application relates to an electrochemomechanical deformation method making said conductive polymer forms deform by electrochemical redox cycle in electrolyte. By applying potential to said conductive polymer forms, excellent electrochemical strain per redox cycle can be obtained. Further, by an electrochemomechanical deformation method making said conductive polymer forms deform, excellent strain per specific time can also be obtained. Although operational electrolyte for electrochemomechanical deformation in which electrochemomechanical deformation of said conductive polymer forms is conducted is not specifically limited, it is preferably liquid including electrolyte in addition to water which is main solvent for easy density control.

Regarding electrochemomechanical deformation method of the invention of the present application, said electrolyte can include at least one compound selected from the group consisting of trifluoromethanesulfonate ions, anions which include a plurality of fluorine atoms bonded to a central atom, and sulfonate salt whose carbon number is not greater than 3. By making said conductive polymer forms deform electrochemically in such electrolyte, said conductive polymer forms are capable of showing even larger electrochemical strain per redox cycle. In addition, salt used in said electrolyte can clearly be used as salt which is included in solid electrolyte in laminates of the present invention and laminates with solid electrolyte which show excellent electrochemical strain per redox cycle can be obtained.

In order to deform said conductive polymer forms, anions which include trifluoromethanesulfonate ions and/or a plurality of fluorine atoms which are bonded to a central atom that are included in electrolyte which is an external environment as operational electrolyte are the same as anions which include trifluoromethanesulfonate ions and/or plurality of fluorine atoms bonded to a central atom as explained in the process for producing above mentioned conductive polymers.

In addition, the invention of the present application may also be electrochemomechanical deformation method which makes conductive polymer forms deform by electrochemical redox reaction in electrolyte, wherein said electrolyte is a solution including sodium chloride as main electrolyte. By including mainly sodium chloride, which is an electrolyte included in living body component, operation is possible where interchangeability of body fluid of a living body and said electrolyte occurs.

Although the temperature of the electrolyte for electrochemomechanical deformation or of solid electrolyte is not specifically limited, in order to make the above mentioned conductive polymers deform electrochemically at higher speed, temperature is preferably 20 to 100°C, and more preferably, 50 to 80 °C.

### (Actuator)

In addition, the invention of the present application relates to an actuator including a moving portion, electrolyte, and counter electrodes, wherein said moving portion includes conductive polymers obtained by the above process for producing conductive polymers. Although said actuator is not specifically limited as long as it includes a moving portion, electrolyte, and counter electrodes as a device configuration, it is preferable that in said actuator, a shaft attached to a moving portion to prevent leakage at the time of operation is packed in a case, or it is preferable that the actuator is provided with a case stretchable depending on operation of a moving portion since it prevents from leakage of the electrolyte and the like.

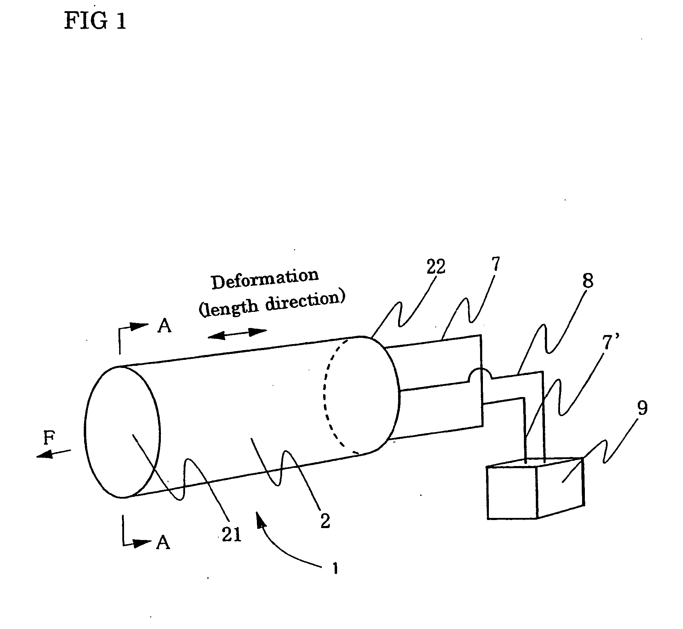

Fig. 1 is a perspective view of the appearance of the actuator of the present invention. Actuator 1 is a cylindrical actuator and an outermost layer is formed by a case formed by flexible materials such a urethane rubber and the like. At bottom portion 22 of actuator 1, lead 8 for imparting potential to operational portion 3 which is inside of the actuator and leads 7 and 7' for providing potential to counter electrodes are provided.

By provision of electricity by power supply 9, and by the voltage application to a moving portion and counter electrodes, moving portion shows electrochemomechanical deformation. By this electrochemomechanical deformation, an end portion of actuator 1 generates strain which is accompanied by the stretch in the length direction. Actuator 1 is capable of generating pressing force F at the time of stretching.

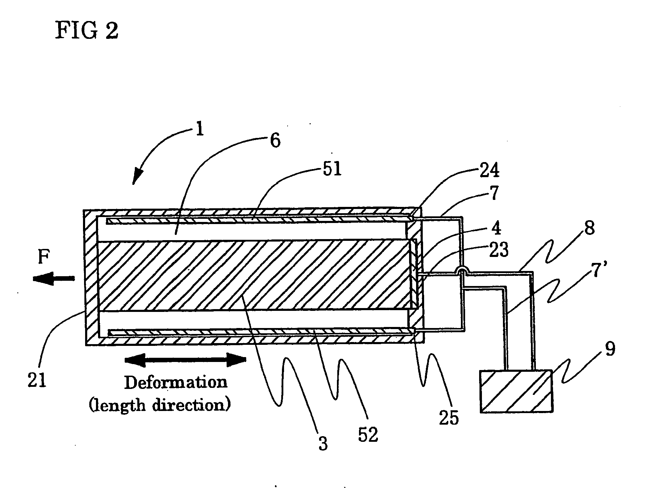

Fig. 2 is a sectional view taken along the line A-A regarding actuator 1 of Fig. 1. Actuator 1 is provided with cylindrical shaped moving portion 3 in the space inside of case 2 molded by flexible materials. In the inside surface of bottom portion 22 of case 2, concave portion 23 is formed. One end portion of moving portion 3 of concave portion 23 is fitted interposing conductive connecting plate 4 therebetween and an operating portion is attached to case 2. In the inside surface of end portion 21 of case 2, by conjugating with other end portion of moving portion 3, column shaped counter electrodes 51, 52 in the vicinity of the internal surface of a side surface of case 2 are attached by respectively fitting to concave portions 24, 25 for fitting counter electrodes. Electrolyte 6 is filled in the internal space excluding counter electrodes 51, 52, and moving portion 3 in the internal space of case 2. Power supply 9 is connected to counter electrodes 51 and 52 interposing leads 7 and 7' therebetween and is connected to conductive connecting plate 4 which contacts with moving portion 3 interposing lead 8 therebetween.

By provision of electricity by power supply 9, voltage can be applied between counter electrodes 51, 52 and moving portion 3, thereby enabling moving portion 3 to show electrochemomechanical deformation. By the stretch of actuator 1, generation of force F at end portion 21 is available and the actuator can preferably be used as artificial muscles.

End portion 21 of actuator 1 may be or need not be conjugated to a tip end of moving portion 3 in the internal surface. In the case where end portion 21 and an end portion of moving portion 3 are not conjugated, in case 2 molded by flexible materials of actuators, by making force orient inside of actuators by using contraction stress, moving portion 3 deforms electrochemically, thereby making end portion 21 stretch following electrochemomechanical deformation of moving portion 3.

Said moving portion is not specifically limited as long as it includes above mentioned conductive polymers and shows electrochemomechanical deformation by voltage application. Particularly, it is preferable that said moving portion shows electrochemical strain of not less than 5% at the time of voltage application. By the stretch of said moving portion of not less than 5% at the time of voltage application, actuator stretching not less than 5% can be obtained and this actuator is preferably used when large electrochemical strain is required, for example, for the use represented by artificial muscles. As said moving portion, conductive materials including for example metal wires or conductive oxides can appropriately be included in addition to dopant in order to lower the resistivity as an operational electrode.

Flexible materials forming case 2 are not specifically limited. Said flexible materials can appropriately be selected depending on stretching ratio of actuators and synthetic resin having the stretching ratio of not less than 5% is preferably used, and synthetic resin having the stretching ratio of not less than 20% is more preferably used. As said flexible materials, for example, silicone resin, urethane resin, silicone rubber, or urethane rubber can be used. In addition, since said flexible materials also have functions of preventing leakage of electrolyte out of actuators, they preferably have resistance to solvents and silicone resin, urethane resin, silicone rubber, and urethane rubber can preferably be used.

In addition, since actuator 1 is provided with a structure in which moving portion is closely packed by case 2, compared with a structure such as rod-shaped structure in which force transmitting means penetrates a case, no leakage of electrolyte occurs even for a long time use, which makes actuator 1 excellent for the use of mechanical parts of artificial muscles.

Shapes of actuators of the present invention are not specifically limited. Although said actuator is formed as cylindrical shape in Fig. 1, the most suitable shapes can be selected depending on the use. As shapes of said actuators, in addition to cylindrical shapes, polygonal shapes such as prismatic or hexagonal, conical shapes, plate shapes, and rectangular solid shapes can be formed depending on the use situation.

Further, moving portions provided inside of actuators of the present invention are not limited to cylindrical shapes and appropriate shapes can be made depending on surface configurations of actuators including polygonal shapes such as prismatic or hexagonal, conical shapes, and rectangular solid shape. Regarding said moving portions, conductive polymer forms obtained on working electrodes by electrochemomechanical deformation may be used as they are or forms such as laminates and the like may be applied thereby making desired shapes. Further, regarding counter electrodes, they are not limited to column shaped and they may be made into such shapes as plates.

Electrolyte includes in the actuator of the present invention may be liquid or solid electrolyte. When said electrolyte is liquid, although solvent may be water or organic solvent, water solvent is preferable for easy handling since vaporizing speed is comparatively slow and large stretch can be obtained. When said electrolyte is solid, although it may be polymer electrolyte or completely solid electrolyte, polymer gel electrolyte is preferable for large ionic conductivity in electrolyte. As gel used for said polymer gel electrolyte, polyacrylamide, polyethylene glycol, and agar are preferably used for easy preparation of polymer gel electrolyte by compounding with aqueous solution electrolyte. It is preferable that said electrolyte includes at least one compound selected from the group consisting of trifluoromethanesulfonate ions, anions which include a plurality of fluorine atoms bonded to a central atom, and sulfonate salt whose carbon number is not greater than 3, since actuators can generate even larger deformations per redox cycle.

In addition, in said actuators, as moving portions and electrolyte, laminates of conductive polymer-containing layer and solid electrolyte layer can also be used. Said laminates can be used for the use which requires large stretching due to large stretching of conductive polymers in the layer by using layers which include conductive polymer obtained by process for producing above mentioned conductive polymer in the conductive polymer-containing layer. In addition, counter electrodes may be so installed that the voltage can be applied between counter electrodes and conductive polymer-containing layer interposing said solid electrolyte therebetween and the place for installation thereof is not specifically limited.

Since said actuator can obtain large electrochemical strain by including above mentioned conductive polymer, in the internal surface of a moving portion, it can preferably be used as artificial muscles in which large strain is required other than for the uses such as switches, sensors, and the like which can be used even with small strain. In other words, although conventional actuators using conductive polymers in a driving part are only applicable for uses in which the strain required is small, the actuators of the present invention can be applied for use in artificial muscles which requires large strain. Said actuators can be used as linear actuators and they can be used as driving devices by attaching members for transmitting force including for example metal wires interposing connectors for driving in end portion 21 of actuator 1 in Fig. 1, for example. In addition, by pressing end portion 21 on a material to be controlled, actuators of the present invention can be used as pressing devices. Since actuators of the present invention are actuators in which conductive polymers are driven by electricity, they are silent at the time of driving and therefore, they are preferable as driving parts or pressing parts in devices for indoor use. In addition, since the actuators employ few metal parts, they are light weight compared to conventional linear actuators and therefore, they can preferably be used as driving devices of positioning devices, posture control devices, elevating devices, carrier devices, moving devices, regulating devices, adjusting devices, guiding devices, and joint devices.

### (Use)

The present invention is useful for artificial muscles, robot arms, power suits, artificial arms, and artificial legs, and for medical instruments such as tweezers, scissors, forceps, snares, laser knives, spatulas, clips which are used in microsurgery techniques, various kinds of sensors or tools for mending which test or mend, industrial devices such as health appliances, hygrometers, humidity controlling devices, soft manipulators, in-water valves or soft carrying devices, hobby goods such as in-water mobiles including gold fish mobiles, moving feeds for fishing, and propulsion fins, which are used in water as well.

In conductive polymer forms, for example, such forms that are not coated with resins, can be used as actuators which can show rectilinear strain in electrolyte. In laminates, for example, when the intermediate layer is a conductive polymer layer and one layer or both layers of upper and lower layers is/are solid electrolyte layer(s) that has/have the equivalent or larger electrochemical strain at the time of electrochemomechanical deformation of conductive polymer layers, such layer(s) can be used as actuators which show rectilinear strain. In addition, in laminates, for example, when one layer of upper and lower layers making conductive polymer layer a middle layer is/are solid electrolyte layer(s) that has/have smaller electrochemical strain at the time of electrochemomechanical deformation of conductive polymer layer or are resin layers, since solid electrolyte layers or resin layers deform less compared with conductive polymer layers, they can be used as actuators generating bent strain. Actuators which generate rectilinear deformation or bent deformation can be used as a driving part generating a rectilinear driving force or as a driving part generating a driving force for moving track shaped rails composed of circular arc portions. Further, said actuators can be used as pressing parts which shows rectilinear movement.

Actuators of the present invention can preferably be used as driving parts which generate rectilinear driving force, as driving parts which generate driving force for moving on track shaped rails composed of circular arc portions, or as pressing parts moving in a rectilinear manner or in a curved manner in OA apparatuses, antennae, seating devices such as beds or chairs, medical apparatuses, engines, optical equipments fixtures, side trimmers, vehicles, elevating machines, food processing devices, cleaning devices, measuring instruments, testing devices, controlling devices, machine tools, process machinery, electronics devices, electronic microscopes, electric razors, electric tooth brushes, manipulators, masts, play game devices, amusement devices, simulation devices for automobiles, holding devices for vehicle occupants, and expanding devices for accessories in aircrafts. Said actuators can be used as driving parts which generate rectilinear driving force, as driving parts which generate driving force for moving on track shaped rails composed of circular arc portions, or as pressing parts moving in a rectilinear manner in, for example, valves, brakes, and lock devices used for machinery as a whole including the above mentioned instruments such as OA apparatus, or measuring instruments. Further, other than said devices, instruments and machines, in mechanical components as a whole, said actuators can preferably be used as driving parts of positioning devices, driving parts of posture control devices, driving parts of elevating devices, driving parts of carriers, driving parts of moving devices, driving parts of regulating devices for the content amount, directions, driving parts of adjusting devices of axes, driving parts of guiding devices, and as pressing parts of pressing devices. In addition, said actuators, as driving parts in joint devices, can preferably be used for driving parts which impart revolving movement to joint portions or joints where direct driving is applicable such as joint intermediate members.

Actuators of the present invention can preferably be used, for example, as driving parts of ink jet parts in ink jet printers such as printers, for example for CAD, driving parts for displacing the direction of optical axis of said optical beam in the printer, head driving parts of disc drive devices such as external storage devices, and as driving parts of pressing contact force regulating means of paper in feeders of image forming devices which include printers, copying machines, and facsimiles.

Actuators of the present invention can preferably be used for driving parts of a drive mechanism relocating power feeding portions measuring portions making high frequency power feeding portion such as antennae shared between the frequencies for ratio astronomy move to second focul point, and driving parts for lifting mechanism in masts used for example for vehicle-loaded pneumatic operating deformable masts (telescoping masts) or antennae.

Actuators of the present invention can preferably be used as driving parts of massaging parts of chair-shaped massagers, driving parts of nursing beds or medical beds, driving parts of posture control devices of electrically reclining chairs, driving parts of stretching rods controlling sitting up and down movement of backrest and ottoman of reclining chairs used for massager or comfort chairs, driving parts used for backrests for reclining chairs in nursing beds or leg rests in furniture on which people place some body portions or driving parts used for rotation drive of nursing beds, and driving parts for controlling posture of uprising chairs.

Actuators of the present invention can preferably be used for driving parts of testing devices, driving parts of pressure measuring devices, for example for blood pressure, used for external blood treatment apparatus, driving parts for catheters, endoscopes or tweezers, driving parts of cataract operation devices using ultrasonic, driving parts of movement devices such as jaw movement devices, driving parts of means for relatively deforming members of chassis of hoists for sickly weak people, and driving parts for elevation, moving or posture control of nursing beds.

Actuators of the present invention can preferably be used for, for example, driving parts of vibration-control devices for decreasing vibration transmitted from vibration generating parts such as engines to vibration receivers such as frames, driving parts of valve train devices for intake and exhaust valves of internal combustion engine, driving parts of fuel-injection devices of engines, and driving parts of fuel-providing systems of engines such as diesel engines.

Actuators of the present invention can preferably be used for, for example, driving parts of calibration devices of imaging devices with compensation function for blurring of images due to hand movement, driving parts of lens driving mechanism of lens for, for example, home video camera, driving parts of driving mechanism of mobile lenses of optical devices such as still cameras or video cameras, driving parts of automatic focus parts of cameras, driving parts of lens-barrel used for image-taking devices of video cameras, driving parts of automatic guiders which take in the light of optical telescopes, driving parts of lens driving mechanism or lens-barrel of optical devices having two optical systems such as stereoscopic cameras or binoculars, driving parts or pressing parts providing compressing force to fibers of wavelength conversion of fiber-type wavelength tunable filters used for optical communication, optical information processing and for optical measuring, driving parts of optical axis alignment devices, and driving parts of shutter mechanism of cameras.

Actuators of the present invention can preferably be used for, for example, pressing parts of fixtures for caulking hose clips to hose bodies.

Actuators of the present invention can preferably be used for, for example, driving parts of coil springs of automobile suspensions, driving parts of fuel filler lid openers which unlock fuel filler lid of vehicles, driving parts of stretching and retraction of bulldozer blades, driving parts of driving devices for changing gear ratios of automotive transmissions automatically, or for disengaging and engaging clutches automatically.

Actuators of the present invention can preferably be used for, for example, driving parts of elevating devices of wheel chairs with seat plate elevation devices, driving parts of elevation devices for eliminating the level difference, driving parts of elevation transfer equipment, driving parts for elevating medical beds, electric beds, electric table, electric chairs, nursing beds, elevation tables, CT scanners, cabin tilt devices for trucks or lifters, each kind of elevation machine devices and loading and uploading devices of special vehicles for carrying heavy materials.

Actuators of the present invention can preferably be used for, for example, driving parts of discharge amount controlling mechanism such as nozzle devices for food discharge used in food processing devices.

Actuators of the present invention can preferably be used for, for example, driving parts for elevating a carriage of cleaning devices or cleaning parts.

Actuators of the present invention can preferably be used for, for example, driving parts of measuring parts of three dimensional measuring devices measuring surface shape, driving parts of stage devices, driving parts of sensor parts of such systems as detecting operating characteristics of tires, driving parts of initial speed-imparting devices of evaluation equipment of impact response of force sensors, driving parts of piston driving devices of piston cylinders of devices for testing water-permeability hole, driving parts for aiming in the direction of elevation angles in condensing and tracking type power generating equipments, driving parts of vibrating devices of tuning mirrors of sapphire laser wavelength switching mechanism for measuring devices which include measuring devices for gas concentration, driving parts of XY θ table when alignment is required in testing devices of printed circuit boards and in testing devices of flat panel displays such as liquid crystals or PDPs, driving parts of adjustable aperture devices used in charged particles beam systems such as electronic beam (E beam) systems or focused ion beam (FIB) systems, driving parts of supporting devices of elements under test or sensing parts in flatness measuring devices, and driving parts of precisely positioning devices such as microscopic device assembly, semi-conductor photolithography machines, semi-conductor inspecting devices, or three dimensional measuring devices.

Actuators of the present invention can preferably be used for, for example, driving parts of electric razors, and driving parts of electric toothbrushes.

Actuators of the present invention can preferably be used for, for example, driving parts of imaging devices of three dimensional objects, driving parts of optical devices for optical system adjusting focal depth for reading out commonly used for CDs and DVDs, driving parts of variable mirrors capable of easily varying focal positions by changing the shape of a surface subject to drive by plural of actuators as active curved surfaces to approximately from a desirable curved surface, driving parts of disc devices capable of moving move units in a rectilinear manner having at least one magnetic head such as optical pick up devices, driving parts of head load mechanism of magnetic tape head actuator assembly such as linear tape storage systems, driving parts of image-forming devices applied for electronograph copying machines, printers or facsimiles, driving parts of loaded members such as magnetic head members, driving parts of optical disc exposure devices which drive and control focusing lenses in the direction of optical axis, driving parts of head driving means which drive optical head, driving parts of information recording and playing devices which record information on record media or play information recorded on record media, and driving parts for switching operations of circuit breaker (circuit breaker for power distribution).

Actuators of the present invention can preferably be used for, for example, driving parts of press molding and vulcanizing devices for rubber compositions, driving parts of parts arrangement devices which arrange delivered parts in single rows or in single layers, or arrange said parts in desired posture, driving parts of compression molding devices, driving parts of holding mechanism of welding devices, driving parts of bag filling and packaging machines, driving parts of machine tools such as machining centers, molding machines such as injection molding machines or press machines, driving parts of fluid coating devices such as printing devices, coating devices, or lacquer spraying devices, driving parts of manufacturing devices which manufacture for example camshafts, driving parts of hoisting devices of covering members, driving parts of for example selvedge control elements in shuttle-less looms, driving parts of needle drive systems of tufting machines, looper driving systems, and knife driving systems, driving parts of cam grinders or polishing devices which polish parts such as ultraprecision machining tools, driving parts of break devices of harness frames of looms, driving parts of opening devices which form opening portions of warp thread for weft thread insert in looms, driving parts of peeling devices of protection sheets of semi-conductor substrates, driving parts of threaders, driving parts of assembly devices of electron guns for CTR, driving parts of linear control devices with shifter fork drive selection of Torchon lace machines for manufacturing Torchon lace having applied uses for welt for clothes, table cloths, or sheet coverings, driving parts of horizontal moving mechanisms of anneal window driving devices, driving parts of support arms of glass melting kiln forehearth, driving parts of making forward and backward movement for rack of exposure devices of fluorescent screen forming methods of color TV tubes, driving parts of torch arms of ball bonding devices, driving parts of bonding heads in XY directions, driving parts of mounting processes of parts or measuring inspection processes of parts in mounting chip parts or measuring using probes, elevation driving parts of cleaning supports of board cleaning devices, driving parts of making probe heads scanning on glass board forward or backward, driving parts of positioning devices of exposure devices which transcribe patterns on boards, driving parts of microscopic positioning devices with sub micron orders in the field of high precision processes, driving parts of positioning devices of measurement devices of chemical mechanical polishing tools, driving parts for positioning stage devices preferable for exposure devices or scanning exposure devices used at the time of manufacturing circuit devices such as conductor circuit elements or liquid crystal display elements in lithography processes, driving parts of means of carrying works or positioning works, driving parts for positioning or carrying such as reticle stages or wafer stages, driving parts of stage devices for precisely positioning in chambers, driving parts of positioning devices of work pieces or semi-conductor wafers in chemical mechanical polishing systems, driving parts of stepper devices of semi-conductors, driving parts of devices precisely positioning in guiding stations of processing machines, driving parts of vibration-control devices of passive vibration-control and active vibration-control types for each kind of machine represented by machine tools and the like such as NC machines or machining centers, or steppers in IC industry, driving parts displacing reference grids board of light beam scanning devices in the direction of optical axis of said light beam in exposure devices used for lithography process for manufacturing semi-conductor elements or liquid crystal elements, and driving parts of transfer devices transferring to item processing units in the transverse direction of conveyors.

Actuators of the present invention can preferably be used for, for example, driving parts of positioning devices of probes of scanning probe microscopes such as electron microscopes, and driving parts of positioning of micro-motion devices for sample in electron microscopes.

Actuators of the present invention can preferably be used for, for example, driving parts of joint mechanisms represented by for example wrists of robot arms in robots including auto welding robots, industrial robots, robots for nursing care or manipulators, driving parts of joint other than direct drive type, fingers of robots, driving parts of motion converting mechanisms of slide retractable zipper devices used for example for hands of robots, driving parts of micro manipulators for operating microscopic objects in any state in cell minute operations or in assembly operation of microscopic parts, driving parts of artificial limbs such as electric artificial arms having plural of fingers which can freely open and close, driving parts of robots for handling, driving parts of assistive devices, and driving parts of power suits.

Actuators of the present invention can preferably be used for, for example, pressing parts of the devices pressing upper rotary blades or lower rotary blades of side trimmers.

Actuators of the present invention can preferably be used for, for example, driving parts of generators in play devices such as for pachinko games, driving parts of amusement devices such as dolls or pet robots, and driving parts of simulation devices of those for automobiles.

Actuators of the present invention can preferably be used for, for example, driving parts of valves used for machines in general including the above instruments and for example, said actuators can preferably be used for driving parts of valves of re-condensers of vaporized helium gas, driving parts of bellows type pressure sensitive valves, driving parts of opening devices which drive harness frames, driving parts of vacuum gate valves, driving parts of control valves of solenoid operations for liquid pressure systems, driving parts of valves with movement transmitting devices using pivot levers built in, driving parts of valves of movable nozzles of rockets, driving parts of suck back valves, and driving parts of regulator valves.

Actuators of the present invention can preferably be used for, for example, pressuring parts of brakes used for machines in general including the above mentioned instruments, and pressuring portions of control devices which are preferably used for brakes for emergency, security or stationary, and pressuring portions of brake structures and brake systems.

Actuators of the present invention can preferably be used for, for example, pressuring portions of lock devices used for machines in general including the above mentioned instruments and for example, pressuring portions of mechanical lock devices, pressuring parts of steering lock devices for vehicles, pressuring portions of power transmission devices which have both load shedding mechanisms and connection releasing mechanisms.

### (EXAMPLES)

Hereinafter, examples of the present invention and comparative examples are shown, however, the invention of the present application is not limited to what is shown hereinafter.

### (Example 1)

Electrolyte was prepared by dissolving monomer and salt of dopant ion stated in table 1 into a solvent stated in table 1 by known stirring method thereby preparing 0.25 mol/l monomer of conductive polymers, making concentration of dopant salt that of table 1.

For this electrolyte, an ITO glass electrode was used as the working electrode and a Pt electrode was used as the counter electrode and electrochemical polymerization was conducted by a constant current method in which polymerization current density is the value shown in table 1. By this electrochemical polymerization, a conductive polymer film of Example 1 having conductivity and a film thickness shown in table 1 was obtained.

### (Examples 2 to 40 and Examples 44 and 45)

Conductive polymer films of Examples 2 to 40 and Examples 44 and 45 were obtained by the same method of Example 1 except that the present Example employed the condition of electrochemical polymerization stated in tables 1 to 6. In addition, in Example 15, regarding monomer of conductive polymers, the mixture ratio of pyrrole and 3-methylthiophene was 1/1 (mol/mol).

### (Example 41)

A conductive polymer film of Example 41 was obtained by the same method of Example 1 except that the present Example employed the condition of electrochemical polymerization stated in table 5 and that the present Example employed a Ti electrode which is a metal electrode as the working electrode. In addition, as the metal electrode of the present application, a commercially available metal electrode was used.

### (Example 42)

A conductive polymer film of Example 42 was obtained by the same method of Example 1 except that the present Example employed the condition of electrochemical polymerization stated in table 5 and that the present Example employed a Ni electrode which is metal electrode as the working electrode.

### (Example 43)

A conductive polymer film of Example 43 was obtained by the same method of Example 1 except that the present Example employed the condition of electrochemical polymerization stated in table 5 and that the present Example employed a Ni electrode which is metal electrode as the working electrode:

### (Comparative Examples 1 to 4)

Conductive polymer films of Comparative Examples 1 to 4 were obtained by the same method of Example 1 except that the present Example employed the condition of electrochemical polymerization stated in tables 5 and 6.

### (Example 46)

Electrolyte was prepared by dissolving salt of dopant ion stated in table 7 into a solvent stated in table 1 by known stirring method thereby preparing electrolyte including pyrrole which is monomer with concentration of 0.25 mol/l and including dopant salt as concentration of table 1. Using this electrolyte, a Ni electrode was used as the working electrode and a Pt electrode was used as the counter electrode and electrochemical polymerization was conducted by a constant current method of polymerization current density stated in table 1. By this electrochemical polymerization, a conductive polymer film of Example 46 shown in Example 46 was obtained.

### (Examples 47 to 62)

Conductive polymer films of each Example were obtained by the same method of Example 46 except that the present Examples employed the condition of electrochemical polymerization stated in tables 7, 8 and 10.

### (Comparative Examples 5 to 16)

Conductive polymer films of Comparative Examples 5 to 16 were obtained by the same method of Example 46 except that the present Examples employed the condition of electrochemical polymerization stated in tables 9 and 10 and that a glass electrode was used as the working electrode.

**Table 1**

| | | Example | | | | | | | |
|---|---|---|---|---|---|---|---|---|---|
| | | 1 | 2 | 3 | 4 | 5 | 6 | 7 | 8 |
| Condition of electrochemical polymerization | Monomer (0.25mol/l) | Pyrrole | Pyrrole | Pyrrole | Pyrrole | Pyrrole | Pyrrole | Pyrrole | Pyrrole |
| | Dopant salt A (mol /l) | 0.5 | 0.5 | 0.5 | 0.5 | 0.5 | 0.5 | 0.5 | |
| | Dopant salt B (mol /l) | | | | | | | | 0.5 |
| | Dopant salt C (mol /l) | | | | | | | | |
| | Dopant salt D (mol /l) | | | | | | | | |
| | Dopant salt E (mol /l) | | | | | | | | |
| | Solvent | PC | PC | PC | EC/PC=1/2 | EC/PC=1/2 | γ-BL | EC/PC=1/2 | EC/PC=1/2 |
| | Current density of polymerization (m A /cm²) | 1 | 1 | 1 | 1 | 1 | 1 | 0.2 | 1 |
| Film property | Conductivity(S/cm) | 29 | 29 | 29 | 43 | 43 | 20 | 9 | 34 |
| | Film thickness(*µ* m) | 50 | 50 | 50 | 36 | 36 | 51 | 57 | 36 |
| Electrochemo mechanical deformation | Operation electrolyte | NaPF₆ | CF₃SO₃Na | NaBF₄ | NaPF₆ | LiAsF₆ | NaPF₆ | NaPF₆ | NaPF₆ |
| | Electrochemical strain(%) | 6.3 | 5.2 | 5.0 | 8.7 | 7.4 | 6.9 | 8.8 | 8.1 |

**Table 2**

| | | Example | | | | | | | | |
|---|---|---|---|---|---|---|---|---|---|---|
| | | 9 | 10 | 11 | 12 | 13 | 14 | 15 | 16 | 17 |
| Condition of electrochemical polymerization | Monomer species (0.25mol/l) | Pyrrole | Pyrrole | Pyrrole | Pyrrole | Pyrrole | Pyrrole +3-Methylthiophene | Pyrrole | Pyrrole | Pyrrole |
| | Dopant salt A (mol /l) | | | | | | | 0.5 | | |
| | Dopant salt B (mol /l) | | | | | | | | | |
| | Dopant salt C (mol /l) | 0.5 | 0.5 | 0.5 | 0.5 | | 0.5 | | 0.5 | 1.0 |
| | Dopant salt D (mol /l) | | | | | 0.5 | | | | |
| | Dopant salt E (mol /l) | | | | | | | | | |
| | Solvent | EC/PC=1/2 | MO | DEC | DMC | EC/PC=1/2 | PC | EC/PC=1/2 | DME | DME |
| | Current density of polymerization (m A /cm²) | 1 | 1 | 1 | 1 | 1 | 1 | 0.2 | 1 | 0.2 |
| Film property | Conductivity (S/cm) | 17 | 13 | 28 | 13 | 13 | 22 | 29 | 40 | 34 |
| | Film thickness (*µ* m) | 68 | 90 | 34 | 26 | 126 | 46 | 50 | 34 | 53 |
| Electrochemomechanical deformation | Operation electrolyte | NaPF₆ | NaPF₆ | NaPF₆ | NaPF₆ | NaPF₆ | NaPF₆ | NaCl | NaPF₆ | NaPF₆ |
| | Electrochemical strain (%) | 7.8 | 5.0 | 8.5 | 7.5 | 5.1 | 5.3 | 3.1 | 10.1 | 10.3 |

**Table 3**

| | | Example | | | | | | | | | |
|---|---|---|---|---|---|---|---|---|---|---|---|
| | | 18 | 19 | 20 | 21 | 22 | 23 | 24 | 25 | 26 | 27 |
| Condition of electrochemical polymerization | Monomer species (0.26mol/l) | Pyrrole | Pyrrole | Pyrrole | Pyrrole | Pyrrole | Pyrrole | Pyrrole | Pyrrole | Pyrrole | Pyrrole |
| | Dopant salt A (mol /l) | | | | | | | | | | |
| | Dopant salt B (mol /l) | | | | | | | | | | |
| | Dopant salt C (mol /l) | 0.5 | 0.5 | 0.5 | 0.5 | 0.5 | 0.5 | 0.5 | 0.5 | 1.0 | 0.5 |
| | Dopant salt D (mol /l) | | | | | | | | | | |
| | Dopant salt E (mol /l) | | | | | | | | | | |
| | Solvent | THF | AcEt | Ac-n-Bt | Ac-t-Bt | EG | PEG/PC= 4/1 | SF | DO | DAE | NM |
| | Current density of polymerization (m A /cm²) | 0.2 | 0.2 | 0.2 | 0.2 | 0.2 | 0.2 | 0.2 | 0.2 | 0.2 | 0.2 |
| Film property | Conductivity (S/cm) | 2.4 | 34 | 50 | 59 | 17 | 83 | 11 | 121 | 63 | 0.2 |
| | Film thickness (*µ* m) | 91 | 50 | 35 | 14 | 47 | 49 | 42 | 13 | 26 | 112 |
| Electrochemo mechanical deformation | Operation electrolyte | NaPF₆ | NaPF₆ | NaPF₆ | NaPF₆ | NaPF₆ | NaPF₆ | NaPF₆ | NaPF₆ | NaPF₆ | NaPF₆ |
| | Electrochemical strain (%) | 7.3 | 8.4 | 9.2 | 10.2 | 5.7 | 8.6 | 8.0 | 9.1 | 9.6 | 6.2 |

**Table 4**

| | | Example | | | | | | | |
|---|---|---|---|---|---|---|---|---|---|
| | | 28 | 29 | 30 | 31 | 32 | 33 | 34 | 35 |
| Condition of electrochemical polymerization | Monomer species (0.25mol/l) | Pyrrole | Pyrrole | Pyrrole | Pyrrole | Pyrrole | Pyrrole | Pyrrole | Pyrrole |
| | Dopant salt A (mol /l) | | | | 0.1 | 0.5 | 0.1 | 0.5 | |
| | Dopant salt B (mol /l) | | | | | | | | |
| | Dopant salt C (mol /l) | 0.5 | 0.5 | 0.5 | | | | | 0.5 |
| | Dopant salt D (mol /l) | | | | | | | | |
| | Dopant salt E (mol /l) | | | | | | | | |
| | Solvent | HxOH | OtOH | EC/PC=1/2 | EC/PC=1/1 | EC/PC=1/1 | EC/PC=1/1 | EC/PC=1/2 | DME |
| | Current density of polymerization (m A /cm²) | 0.2 | 0.2 | 0.2 | 1 | 1 | 1 | 1 | 1 |
| Film property | Couductivity(S/cm) | 50 | 69 | 29 | 42 | 10 | 33 | 29 | 40 |
| | Film thickness (*µ* m) | 31 | 19 | 50 | 32 | 94 | 23 | 50 | 34 |
| Electrochemo mechanical deformation | Operation electrolyte | NaPF₆ | NaPF₆ | TEAPF₆ | NaPF₆ | NaPF₆ | NaPF₆ | EtSO₃Na | LiAsF₆ |
| | Electrochemical strain Electrochemical (%) | 9.1 | 10.3 | 6.7 | 7.6 | 8.3 | 8.9 | 6.4 | 9.0 |

**Table 5**

| | | Example | | | | | | | | Comparative Example | |
|---|---|---|---|---|---|---|---|---|---|---|---|
| | | 36 | 37 | 38 | 39 | 40 | 41 | 42 | 43 | 1 | 2 |
| Condition of electrochemical polymerization | Monomer species (0.25mol/l) | Pyrrole | Pyrrole | Pyrrole | Pyrrole | Pyrrole | Pyrrole | Pyrrole | Pyrrole | Pyrrole | Pyrrole |
| | Dopant salt A (mol /l) | | | | | | | | 0.5 | | |
| | Dopant salt B (mol /l) | | | | | | | | | | |
| | Dopant salt C (mol/l) | 0.5 | 0.5 | 0.5 | 0.5 | 0.5 | 0.5 | 0.5 | | | |
| | Dopant salt D (mol/l) | | | | | | | | | | |
| | Dopant salt E (mol/l) | | | | | | | | | 0.5 | 0.5 |
| | Solvent | AN | NB | MeB | PhEt | DCM | MeB | BuB | MeB | H₂O | H₂O |
| | Current density of polymerization (m A /cm²) | 0.2 | 0.2 | 0.2 | 0.2 | 0.2 | 0.2 | 0.2 | 0.2 | 1 | 1 |
| Film property | Conductivity (S/cm) | 78 | 46 | 53 | 35 | 3 | 112 | 62 | 113 | 42 | 42 |
| | Film thickness (*µ* m) | 13 | 21 | 24 | 44 | 24 | 18 | 15 | 31 | 36 | 36 |
| Electrochemo mechanical deformation | Operation electrolyte | NaPF₆ | NaPF₆ | NaPF₆ | NaPF₆ | NaPF₆ | NaPF₆ | NaPF₆ | NaPF₆ | NaCl | NaPF₆ |
| | Electrochemical strain (%) | 8.1 | 9.7 | 11.4 | 10.0 | 8.6 | 12.4 | 15.1 | 10.3 | 1.3 | 1.7 |

**Table 6**

| | | Example | | Comparative Example | |
|---|---|---|---|---|---|
| | | 44 | 45 | 3 | 4 |
| Condition of electrochemical polymerization | Monomer (0.25mol/l) | Pyrrole | Pyrrole | Pyrrole | Pyrrole |
| | Dopant salt A (mo l/l) | 0.5 | 0.5 | | |
| | Dopant salt B (mol /l) | | | | |
| | Dopant salt C (mol /l) | | | | |
| | Dopant salt D (mol /l) | | | | |
| | Dopant salt E (mol /l) | | | 0.5 | 0.5 |
| | Solvent | EC/PC=1/2 | EC/PC=1/2 | H₂O | H₂O |
| | Current density of polymerization (m A /cm²) | 0.2 | 1 | 1 | 1 |
| Film property | Conductivity(S/cm) | 29 | 43 | 42 | 42 |
| | Film thickness *µ* m) | 50 | 36 | 36 | 36 |
| Electrochemo mechanical deformation | Operation electrolyte | NaCl | NaPF₆ | NaCl | NaPF₆ |
| | Electrochemical strain per specific time (%/20seconds) | 1.7 | 3.9 | 0.4 | 0.4 |

**Table 7**

| | | Example | | | | | | | |
|---|---|---|---|---|---|---|---|---|---|
| | | 46 | 47 | 48 | 49 | 50 | 51 | 52 | 53 |
| Condition of electrochemical polymerization | Dopant salt C (mol/l) | 0.5 | | 0.5 | 0.5 | 0.5 | 0.5 | 0.5 | 0.5 |
| | Dopant salt A (mol /l) | | 0.5 | | | | | | |
| | Metal species of electrodes | Ni | Ni | Ni | Ti | Pt | Ni | Ni | Ti |
| | Solvent | PC | PC | MeB | MeB | MeB | BuB | DME | DME |
| | Current density of polymerization (m A /cm²) | 0.2 | 0.2 | 0.2 | 0.2 | 0.2 | 0.2 | 0.2 | 0.2 |
| Film Property | Conductivity (S/cm) | 54 | 19 | 112 | 87 | 55 | 30 | 22 | 124 |
| | Film thickness (*µ* m) | 44 | 37 | 18 | 32 | 26 | 8 | 13 | 31 |
| Electrochemomechanical deformation | Electrochemical strain (%) | 3 | 5 | 5 | 5 | 5 | 5 | 3 | 5 |
| | Electrochemical stress (MPa) | 4.5 | 3.9 | 10.5 | 8.7 | 6.8 | 15.6 | 4.7 | 6.1 |
| Comparison with when non-metal electrodes are used | Corresponding comparative example | Comparative example 5 | Comparative example 6 | Comparative example 7 | Comparative example 7 | Comparative example 7 | Comparative example 8 | Comparative example 10 | Comparative example 9 |
| | Ratio of electrochemical stress | 4.5 | 3.3 | 3.1 | 2.6 | 2.0 | 4.1 | 3.6 | 6.8 |

**Table 8**

| | | Example | | | | |
|---|---|---|---|---|---|---|
| | | 54 | 55 | 56 | 57 | 58 |
| Condition of electrochemical polymerization | Dopant salt C (mol/l) | 0.5 | 0.5 | 0.5 | 0.5 | 0.5 |
| | Dopant salt A (mol/ l) | | | | | |
| | Metal species of electrodes | Ni | Ti | Ni | Ni | Ni |
| | Solvent | EtPh | EtPh | DCM | MMP | MeSa |
| | Current dersity of polymerization (m A /cm²) | 0.2 | 0.2 | 0.2 | 0.2 | 0.2 |
| Film Property | Conductivity (S/cm) | 27 | 21 | 108 | 50 | 29 |
| | Film thickness(µm) | 18 | 38 | 5 | 30 | 57 |
| Electrochemomechanical deformation | Electrochemical strain (%) | 5 | 5 | 5 | 5 | 5 |
| | Electrochemical stress (MPa) | 5.3 | 5.2 | 3.2 | 6.8 | 8.2 |
| Comparison with when non-metal electrodes are used | Corresponding comparative example | Comparative example 11 | Comparative example 11 | Comparative example 12 | Comparative example 13 | Comparative example 14 |
| | Ratio of electrochemical stress | 2.1 | 2.1 | 2.7 | 10.5 | 2.8 |

**Table 9**

| | | Example | | | | | | | | | |
|---|---|---|---|---|---|---|---|---|---|---|---|
| | | 5 | 6 | 7 | 8 | 9 | 10 | 11 | 12 | 13 | 14 |
| Condition of electrochemical polymerization | Dopant salt C (mol /l) | 0.5 | | 0.5 | 0.5 | 0.5 | 0.5 | 0.5 | 0.5 | 0.5 | 0.5 |
| | Dopant salt A (mol /l) | | 0.5 | | | | | | | | |
| | Kind of non-Metal electrodes | ITO glass | ITO glass | ITO glass | ITO glass | ITO glass | ITO glass | ITO glass | ITO glass | ITO glass | ITO glass |
| | Solvent | PC | PC | MeB | BuB | DME | DME | EtPh | DCM | MMP | MeSa |
| | Current density of polymerization (m A /cm²) | 0.2 | 0.2 | 0.2 | 0.2 | 0.2 | 0.2 | 0.2 | 0.2 | 0.2 | 0.2 |
| Film Property | Conductivity (S/cm) | 32 | 16 | 53 | 65 | 40 | 40 | 35 | 3 | 55 | 8 |
| | Film thickness(*µ* m) | 51 | 37 | 24 | 23 | 34 | 34 | 44 | 24 | 28 | 18 |
| Electrochemomechanical deformation | Electrochemical strain (%) | 3 | 5 | 5 | 5 | 5 | 3 | 5 | 5 | 5 | 5 |
| | Electrochemical stress (MPa) | 1.0 | 1.2 | 3.4 | 3.8 | 0.9 | 1.4 | 2.5 | 1.2 | 0.6 | 2.9 |

**Table 10**

| | | Example | | | | Comparative Example | |
|---|---|---|---|---|---|---|---|
| | | 59 | 60 | 61 | 62 | 15 | 16 |
| Condition of electrochemical polymerization | Dopant salt C (mol /l) | 0.5 | 0.5 | 0.5 | | | |
| | Dopant salt A (mol /l) | | | | 0.5 | | |
| | Dopant salt F (mol /l) | | | | | 0.5 | |
| | Dopant salt E (mol /l) | | | | | | 0.5 |
| | Metal species of electrodes | Ni | Ti | Ti | Ti | | |
| | Kind of non-Metal electrodes | | | | | ITO | ITO |
| | Solvent | MeB | MeB | DME | MeB | H2O | H2O |
| | Current density of polymerization (m A /cm²) | 0.2 | 0.2 | 0.2 | 0.2 | 1 | 1 |
| Film Property | Conductivity (S/cm) | 112 | 87 | 124 | 113 | 22 | 42 |
| | Film thickness(*µ* m) | 18 | 32 | 31 | 31 | 38 | 36 |
| Electrochemomechanical deformation | Electrochemical stress (MPa) | 17.7 | 18.4 | 14.2 | 13.4 | 0.7 | 3.5 |

For information, in tables 1 to 10, the abbreviation of the column of kinds of dopant salt and of solvent is as follows.
Dopant salt A: TBACF₃SO₃ (tetrabutylammonium trifluoromethanesulfonate)
Dopant salt B: CF₃SO₃Li (lithium trifluoromethanesulfonate)
Dopant salt C: TBABF₄ (tetrabutylammonium tetrafluoroborate)
Dopant salt D: TBAPF₆ (tetrabutylammonium hexafluorophosphate)
Dopant salt E: sodium p-toluenesulfonate
Dopant salt F: sodium benzenesulfonate

For information, in tables 1 to 4, the abbreviation of the column of kinds of dopant salt and of solvent is as follows.

### Solvent;

PC: propylene carbonate
EC: ethylene carbonate
γ -BL: γ -butyrolactone
MO: 3-methyl-2-oxazolidinone
DEC: diethyl carbonate
DMC: dimethyl carbonate
DME: dimethoxy ethane
THF: tetrahydrofuran
AcEt: ethyl acetate
Ac-n-Bt: n-butyl acetate
Ac-t-Bt: t-butyl acetate
EG: ethylene glycol
PEG: polyethylene glycol (molecular weight: 200)
SF: sulfolane
DO: 1,4-dioxane
DAE: 1,2-diacetoxy ethane
NM: nitromethane
HxOH: 1-hexanol
OtOH: 1-octanol
AN: acetonitrile
NB: nitrobenzene
MeB: methyl benzoate
PhEt: diethyl phthalate
DCM: dichloromethane
BuB: butyl benzoate
EtPh: diethyl phthalate
DCM: dichloromethane
MMP: 3-methoxymethylpropionate
MeSa: methyl salicylate

In addition, in tables 1 to 5, when mixed solvent is used, for example, EC/PC=1/2 shows solvent in which the weight ratio of ethylenecarbonate and propylene carbonate is 1 : 2.

### (Evaluation)

Film-like conductive polymer forms obtained by Examples 1 to 43 and Comparative Examples 1 and 2 were retained in electrolyte dissolved in water to make operation electrolyte stated in tables 1 to 5 1 mol/l. Deformation ratio of each retained film-like conductive polymer form per redox cycle was measured respectively by the following measuring method. Results are shown in tables 1 to 5. In addition, strain of film-like conductive polymer forms per specific time obtained in examples 44 and 45, and comparative examples 3 and 4 by the following measuring method is respectively measured. Results of strain per specific time are shown in table 6. For information, conductivity and film thickness of conductive polymer forms of examples 1 to 45 and comparative examples 1 to 4 were measured by known methods.

### (Measuring method of electrochemical strain)

A conductive polymer film obtained by Examples 1 to 43 and Comparative Examples 1 and 2 was used as the working electrode with 15 mm in length and 2 mm in width, and a platinum plate was used as the counter electrode, and in the end portion of each electrode, a working electrode was held in said electrolyte, and was connected to the power supply interposing a lead, thereby measuring the electrochemomechanical deformation (change in length) when potential was cycled (between -0.9 V and + 0.7 V vs. Ag/Ag⁺). Electrochemical strain per redox cycle was defined by dividing the change in length obtained by deformation of a working electrode which was cycled (one redox cycle) by the original length of a working electrode. For information, TEAPF₆ of the operation electrode represents tetraethylammonium hexafluorophosphate and EtSO₃Na represents sodium ethanesulfonate.

### (Electrochemical strain per specific time)

A conductive polymer film obtained by Examples 44 and 45 and Comparative Examples 3 and 4 was used as the working electrode with 15 mm in length and 2 mm in width, and a platinum plate was used as the counter electrode, and in the end portion of each electrode, a working electrode was held in said electrolyte, and was connected to the power supply interposing a lead, thereby measuring the electrochemomechanical deformation (change in length) 20 seconds after potential was applied at +0.9 V v.s. Ag/Ag⁺ or -0.9 V v.s. Ag/Ag⁺). Electrochemical strain per specific time was defined by dividing the change in length 20 seconds after potential was applied by the original length of a working electrode before potential was applied.

### (Electrochemical stress)

A conductive polymer film obtained by Examples 46 to 62 and Comparative Examples 5 to 16 was used as the working electrode with 15 mm in length and 2 mm in width, and a load was suspended in the end portion of each conductive polymer film and each of the other end was held in operation electrolyte and was connected to the power supply interposing a lead, thereby measuring the electrochemomechanical deformation (change in length) when potential was cycled (between -0.9 V and + 0.7 V vs. Ag/Ag⁺). Regarding conductive polymer forms of Examples 46 to 58 and Comparative Examples 5 to 14, by dividing change in electrochemical strain obtained by the contraction of the working electrode which was cycled (one redox cycle) by the original length of the working electrode, electrochemical strain per one redox cycle was defined, which were shown in tables 6 to 9. The electrochemical stress was defined by the weight of a load with these electrochemical strains. As said operation electrolyte, 15 wt % of aqueous solution of sodium hexafluorophosphate was used. In addition, electrochemical stress was measured by measuring electrochemical strain for a loaded weight by changing the weight of said load and by converting the measured values per unit section area. Further, regarding conductive polymer films of Examples 59 to 62 and Comparative Examples 15 and 16, by the same method of measuring electrochemical strain of conductive polymer films of Examples 46 to 62 and Comparative Examples 5 to 16, electrochemical strain was measured and the maximum electrochemical stress of each conductive polymer film was defined and was shown in table 10. For information, the maximum electrochemical stress was measured by increasing the load and within the range of deformation, by the electrochemical stress right before the conductive polymer film was broken by the weight of a load. Conductivity and film thickness of conductive polymer films in Examples 46 to 62 and Comparative Examples 5 to 16 were measured by a known method.

### (Ratio of electrochemical stress when non-metal electrodes are used)

Regarding conductive polymer films of Examples 46 to 58, ratio of ( [electrochemical stress in the Examples] / [electrochemical stress in the Comparative Examples]) was calculated when showing the same electrochemical strain regarding Comparative Examples which correspond to conductive polymer forms produced by the same electrochemical polymerization condition except that ITO glass electrodes which are non-metal electrodes were used as the working electrode. Results were shown in tables 6 to 8.

### (Results)

Conductive polymer films of examples 1 to 45 are conductive polymer films which include conductive polymers as resin components obtained by the first process for producing conductive polymers of the present invention.

Conductive polymer films of Example 15 are conductive polymer films obtained by process for producing conductive polymers by electrochemical polymerization which uses electrolyte in which trifluoromethanesulfonate ion is made to be dopant anion and in which solvents are mixed solvents of ethylene carbonate and propylene carbonate (1:2). In conductive polymer forms of Comparative Example 1, solvent of electrolyte was water and electrochemical polymerization was conducted by electrolyte which includes p-toluensulfonate ion which is conventional dopant. When conductive polymer films of Example 15 were electrochemically deformed with sodium chloride which is a conventional environment as operation electrolyte, as shown in table 2, electrochemical strain was 3.1 %. On the other hand, when conductive polymer films of Comparative Example 1 were electrochemically deformed with sodium chloride which is a conventional environment as operation electrolyte as in Example 15, as shown in table 4, electrochemical strain was 1.3%. In other words, conductive polymer films of Example 15 were capable of showing good deformation with electrochemical strain per redox cycle of about 2.4 times compared with conductive polymer films which include conventional dopant even when in sodium chloride solution which is conventional operational environment.

Regarding Examples 1 to 14 and Examples 16 to 43, when conductive polymer films were deformed by electrochemical redox of one redox cycle, in electrolyte including at least one compound selected from the group composed of trifluoromethanesulfonate ions, anions including a plurality of fluorine atoms bonded to a central atom, and sulfonate salt whose carbon number is not greater than 3 as operation environment, the result was, as shown in tables 1 to 5, electrochemical strains were not less than 5%. On the other hand, regarding Comparative Example 2, when conductive polymer films are conductive polymers in which solvent of electrolyte was water and were polymerized electrochemically with electrolyte including p-toluenesulfonate ion which is conventional dopant and including as operation environment, at least one compound selected from the group, composed of trifluoromethanesulfonate ions, fluorine atoms bonded to a central atom, and sulfonate salt whose carbon number is not greater than 3 by electrochemical redox of one redox cycle, the result was, as shown in table 4, that electrochemical strain was as low as 1.7%. In other words, when the conductive polymer film of the present invention was deformed by electrochemical redox in electrolyte including compounds selected at least one from the group composed of trifluoromethanesulfonate ion, fluorine atoms bonded to a central atom, and sulfonate salt whose carbon number is not greater than 3, electrochemical strains per redox cycle were excellent which were more than about triple of the conventional polymer films.

Conductive polymer films of Examples 44 and 45 were the conductive polymer forms obtained according to the present invention which correspond to Examples 15 and 4, respectively. On the other hand, conductive polymer forms of Comparative Examples 3 and 4 are conductive polymer forms which correspond to Comparative Examples 1 and 2 respectively. In aqueous solution of NaCl which is a conventional operation environment, while electrochemical strain of conductive polymer films of Comparative Example 3 per specific time was 0.4%electrochemical strain of conductive polymer forms of the Example 36 per specific time was 1.7% which was enhanced about four times. In other words, by using conductive polymer forms as in the present invention, fast electrochemomechanical deformation can be realized.

In other words, as operation environment of electrochemomechanical deformation, when electrolyte was so prepared that included at least one compound selected from the group composed of trifluoromethanesulfonate ions, anions including a plurality of fluorine atoms bonded to a central atom, and sulfonate salt whose carbon number is not greater than 3, while electrochemical strain of conductive polymer forms of Comparative Example 4 per specific time was 0.4%, electrochemical strain of conductive polymer films of Example 45 per specific time was 3.9% which was enhanced about ten times. Therefore, by employing electrochemomechanical deformation method of the present invention, fast electrochemomechanical deformation can be realized.

Conductive polymer forms of Examples 46 to 62 are conductive polymer forms which include conductive polymers as a resin component obtained using a metal electrode.

Conductive polymer films of Examples 46 to 58 were conductive polymer forms showing electrochemical strain of 3 to 5%, contraction per redox cycle which had not been obtained by the actuators using conventional conductive polymers and accompanying the large electrochemical stress of 3.9 to 15.6 MPa, and having excellent balance of electrochemical strain and stress. Moreover, since conductive polymer films of Examples 46 to 58 used metal electrodes, compared with the Examples in which non-metal electrodes were used, enhancement of excellent electrochemical stress of 2.0 to 10.5 times as large as the Examples which used non-metal electrodes. Further, regarding Examples 59 to 62, although excellent maximum electrochemical stress of 13.4 to 18.4 MPa was obtained, regarding Comparative Examples 17 and 18, each electrochemical stress was at most 0.7 MPa and 3.5 MPa respectively. For information, the maximum electrochemical stress is a force generated just before conductive polymer forms whose electrochemical strain was measured by changing the weight of a load are cut off by the weight of a load within the range of contraction. In addition, in the above mentioned Examples and Comparative Examples, since deformation was measured in a state where the weight was loaded in the direction of gravitational force, the deformation ratio was so defined to make contraction ratio of a conductive polymer forms.

### Industrial applicability

Conductive polymers of the present invention can deform with excellent electrochemical strain by electrochemomechanical deformation. For this reason, said conductive polymers are excellent in practicality since said conductive polymers can make large movement and are useful for the use of, for example, artificial muscles, robot arms, artificial hands, and actuators. In particular, since conductive polymer forms, laminates, and actuators using conductive polymers obtained as in the present invention can deform with excellent electrochemical strain by electrochemomechanical deformation, they can preferably be used for positioning devices, posture control devices, elevation devices, carrier devices, moving devices, regulating devices, adjusting devices, guiding devices, driving part of joint device and pressing part of pressing device. They are useful in applications which require larger deformation for capability of showing larger electrochemical strain.

In addition, conductive polymer forms which include conductive polymers as resin components may be used in the present invention, as operational environment of electrochemomechanical deformation, by deforming conductive polymer forms by electrochemical redox in electrolyte which include at least one component selected from the group consisting of anions including trifluoromethanesulfonate ion and plurality of fluorine atoms bonding to a central atom as operation environment of electrochemomechanical deformation and sulfonate salt whose carbon number is not greater than 3, about ten times or larger electrochemical strain per specific time is developed compared with conductive polymer forms having conventional electrochemical strain. For this reason, these conductive polymer forms can also be used as driving parts for the use which requires quick response to the order of strain.

Further, conductive polymer forms including conductive polymers as resin components obtained using a metal electrode can show excellent electrochemical strain per redox cycle at the time of electrochemomechanical deformation and can obtain excellent electrochemical stress compared with conventional conductive polymer forms having conventional stretching property. This obtained electrochemical stress shows excellent electrochemical stress which is twice as large compared with conductive polymers obtained by conducting electrochemical polymerization using non-metal electrodes. For this reason, said conductive polymer forms are preferable for the use of actuators including micro machines, artificial muscles, and the like. Further, said conductive polymer forms are preferable for micro machines for satisfactory mechanical strength.

## Claims

1. An electrochemomechanical deformation method which comprises deforming a conductive polymer form by electrochemical redox in electrolyte; said conductive polymer form including, as a resin component, a conductive polymer which has deformation property by electrochemical redox, and which has been prepared by an electrochemical polymerization method which is a polymerization method using electrolyte including organic compounds as solvents, and wherein said organic compounds include in a molecule:
(1) at least one chemical bond species selected from the group consisting of ether bond, ester bond, carbon-halogen bond, and carbonate bond, and/or
(2) at least one functional group selected from the group consisting of hydroxyl group, nitro group, sulfone group, and nitryl group;
and wherein said electrolyte used in said polymerization process includes trifluoromethanesulfonate ions and/or anions in which a plurality of fluorine atoms are bonded to a central atom.

2. An electrochemomechanical deformation method as set forth in claim 1, wherein electrochemomechanical deformation is conducted under temperature environment of not lower than room temperature.

3. An electrochemomechanical deformation method as set forth in claim 1, in which the electrolyte used in said deformation method includes at least one compound selected from the group consisting of compounds containing trifluoromethanesulfonate ions and/oranions in which a plurality of fluorine atoms are bonded to a central atom, and sulfonate salt whose carbon number is not greater than 3.

4. An electrochemomechamical deformation method as set forth in claim 1, in which said electrolyte used in said deformation method includes sodium chloride.

5. An actuator comprising a moving part, a counter electrode, and electrolyte, wherein the moving part has been obtained by a process for producing conductive polymers by an electrochemical polymerization method, wherein said conductive polymers have deformation property by electrochemical redox, in which said electrochemical polymerization method is a polymerization method using electrolyte including organic compounds as solvents, and wherein said organic compounds include in a molecule:
(1) at least one chemical bond species selected from the group consisting of ether bond, ester bond, carbon-halogen bond, and carbonate bond,
and/or
(2) at least one functional group selected from the group consisting of hydroxyl group, nitro group, sulfone group, and nitryl group;
and wherein said electrolyte used in said polymerization process includes trifluoromethanesulfonate ions and/or anions in which a plurality of fluorine atoms are bonded to a central atom.

6. An actuator as claimed in claim 5, wherein the moving part deforms by electrochemical redox and the actuator deforms not less than 3% in the length direction.

7. An actuator as claimed in claim 5, wherein the moving part deforms by electrochemical redox and electrochemical strain of the actuator per redox cycle of 20 seconds is not less than 3% in the length direction.

## Patentansprüche

1. Elektrochemisch-mechanisches Verformungsverfahren, das das Verformen einer leitfähigen Polymerform durch elektrochemische Redox-Reaktion in einem Elektrolyt aufweist, wobei die leitfähige Polymerform als einen Harzbestandteil ein leitfähiges Polymer aufweist, das ein Verformungsvermögen durch elektrochemische Redox-Reaktion aufweist und das durch ein elektrochemisches Polymerisationsverfahren hergestellt worden ist, das ein Polymerisationsverfahren ist, das einen Elektrolyt verwendet, der organische Verbindungen als Lösungsmittel aufweist, und wobei die organischen Verbindungen in einem Molekül Folgendes aufweisen:
(1) zumindest eine chemische Bindungsart, die aus der Gruppe ausgewählt ist, die aus einer Etherbindung, Esterbindung, Kohlenstoff-Halogen-Bindung und Carbonatbindung besteht, und/oder
(2) zumindest eine funktionelle Gruppe, die aus der Gruppe ausgewählt ist, die aus einer Hydroxylgruppe, Nitrogruppe, Sulfongruppe und Nitrylgruppe besteht;
und wobei der bei diesem Polymerisationsverfahren verwendete Elektrolyt Trifluormethansulfonationen und/oder Anionen aufweist, worin mehrere Fluoratome an ein zentrales Atom gebunden sind.

2. Elektrochemisch-mechanisches Verformungsverfahren nach Anspruch 1,
wobei die elektrochemisch-mechanische Verformung in einer Temperaturumgebung erfolgt, die nicht unter Raumtemperatur liegt.

3. Elektrochemisch-mechanisches Verformungsverfahren nach Anspruch 1,
wobei der bei diesem Verformungsverfahren verwendete Elektrolyt zumindest eine Verbindung aufweist, die aus der Gruppe ausgewählt ist, bestehend aus Verbindungen, die Trifluormethansulfonationen und/oder Anionen enthalten, worin mehrere Fluoratome an ein zentrales Atom gebunden sind, und Sulfonatsalz, dessen Kohlenstoffzahl nicht größer als 3 ist.

4. Elektrochemisch-mechanisches Verformungsverfahren nach Anspruch 1,
wobei der bei diesem Verformungsverfahren verwendete Elektrolyt Natriumchlorid aufweist.

5. Betätigungseinrichtung, die ein bewegliches Teil, eine Gegenelektrode und einen Elektrolyt aufweist,
wobei das bewegliche Teil nach einem Verfahren zum Herstellen von leitfähigen Polymeren durch ein elektrochemisches Polymerisationsverfahren erhalten worden ist, wobei die leitfähigen Polymere ein Verformungsvermögen durch elektrochemische Redox-Reaktion aufweisen, wobei das elektrochemische Polymerisationsverfahren ein Polymerisationsverfahren ist, das einen Elektrolyt verwendet, der organische Verbindungen als Lösungsmittel aufweist, und wobei die organischen Verbindungen in einem Molekül Folgendes aufweisen:
(1) zumindest eine chemische Bindungsart, die aus der Gruppe ausgewählt ist, die aus einer Etherbindung, Esterbindung, Kohlenstoff-Halogen-Bindung und Carbonatbindung besteht, und/oder
(2) zumindest eine funktionelle Gruppe, die aus der Gruppe ausgewählt ist, die aus einer Hydroxylgruppe, Nitrogruppe, Sulfongruppe und Nitrylgruppe besteht;
und wobei der bei diesem Polymerisationsverfahren verwendete Elektrolyt Trifluormethansulfonationen und/oder Anionen aufweist, worin mehrere Fluoratome an ein zentrales Atom gebunden sind.

6. Betätigungseinrichtung nach Anspruch 5,
wobei sich das bewegliche Teil durch eine elektrochemische Redox-Reaktion verformt und sich die Betätigungseinrichtung in Längenrichtung um nicht wenige als 3 % verformt.

7. Betätigungseinrichtung nach Anspruch 5,
wobei sich das bewegliche Teil durch eine elektrochemische Redox-Reaktion verformt und die elektrochemische Verformung der Betätigungseinrichtung pro Redox-Zyklus von 20 Sekunden nicht weniger als 3 % in Längenrichtung beträgt.

## Revendications

1. Procédé de déformation électro-chimio-mécanique qui comprend la déformation d'une forme de polymère conducteur par oxydoréduction électrochimique dans un électrolyte ; ladite forme de polymère conducteur incluant, en tant que composant de résine, un polymère conducteur qui a une propriété de déformation par oxydoréduction électrochimique, et qui a été préparé par un procédé de polymérisation électrochimique qui est un procédé de polymérisation utilisant un électrolyte incluant des composés organiques comme solvants, et dans lequel lesdits composés organiques incluent dans une molécule :
(1) au moins une espèce de liaison chimique choisie parmi le groupe constitué d'une liaison éther, d'une liaison ester, d'une liaison carbone-halogène et d'une liaison carbonate,
et/ou
(2) au moins un groupe fonctionnel choisi parmi le groupe constitué d'un groupe hydroxyle, d'un groupe nitro, d'un groupe sulfone et d'un groupe nitrile ;
et dans lequel ledit électrolyte utilisé dans ledit processus de polymérisation inclut des ions et/ou des anions trifluorométhanesulfonate dans lesquels une pluralité d'atomes de fluor sont liés à un atome central.

2. Procédé de déformation électro-chimio-mécanique selon la revendication 1, dans lequel la déformation électro-chimio-mécanique est réalisée sous un environnement à température non inférieure à la température ambiante.

3. Procédé de déformation électro-chimio-mécanique selon la revendication 1, dans lequel l'électrolyte utilisé dans ledit procédé de déformation inclut au moins un composé choisi parmi le groupe constitué de composés contenant des ions et/ou des anions trifluorométhanesulfonate dans lesquels une pluralité d'atomes de fluor sont liés à un atome central, et un sel de sulfonate dont le nombre de carbones n'est pas supérieur à 3.

4. Procédé de déformation électro-chimio-mécanique selon la revendication 1, dans lequel ledit électrolyte utilisé dans ledit procédé de déformation inclut du chlorure de sodium.

5. Actionneur comprenant une pièce en mouvement, une contre-électrode et un électrolyte, dans lequel la pièce en mouvement a été obtenue par un processus pour produire des polymères conducteurs par un procédé de polymérisation électrochimique, dans lequel lesdits polymères conducteurs ont une propriété de déformation par oxydoréduction électrochimique, dans lequel ledit procédé de polymérisation électrochimique est un procédé de polymérisation utilisant un électrolyte incluant des composés organiques comme solvants, et dans lequel lesdits composés organiques incluent dans une molécule :
(1) au moins une espèce de liaison chimique choisie parmi le groupe constitué d'une liaison éther, d'une liaison ester, d'une liaison carbone-halogène et d'une liaison carbonate,
et/ou
(2) au moins un groupe fonctionnel choisi parmi le groupe constitué d'un groupe hydroxyle, d'un groupe nitro, d'un groupe sulfone et d'un groupe nitrile ;
et dans lequel ledit électrolyte utilisé dans ledit processus de polymérisation inclut des ions et/ou des anions trifluorométhanesulfonate dans lesquels une pluralité d'atomes de fluor sont liés à un atome central.

6. Actionneur selon la revendication 5, dans lequel la pièce en mouvement se déforme par oxydoréduction électrochimique et l'actionneur ne se déforme pas de moins de 3 % dans la direction de la longueur.

7. Actionneur selon la revendication 5, dans lequel la pièce en mouvement se déforme par oxydoréduction électrochimique et la contrainte électrochimique de l'actionneur par cycle d'oxydoréduction de 20 secondes n'est pas inférieure à 3 % dans la direction de la longueur.
